(19) Europäisches Patentamt
European Patent Office
Office européen des brevets

(11) Publication number : **0 481 719 A1**

(12) # EUROPEAN PATENT APPLICATION

(21) Application number : **91309469.4**

(22) Date of filing : **15.10.91**

(51) Int. Cl.$^5$ : **A61B 5/00,** G01N 33/00, G01N 21/77

(30) Priority : **15.10.90 US 597816**

(43) Date of publication of application : **22.04.92 Bulletin 92/17**

(84) Designated Contracting States : **AT BE CH DE DK ES FR GB GR IT LI LU NL SE**

(71) Applicant : **PURITAN-BENNETT CORPORATION**
**9401 Indian Creek Parkway, Overland Park**
**Kansas City, Kansas (US)**

(72) Inventor : **Nelson, Alan**
**11151 Capilla Road**
**San Diego, CA 92126 (US)**
Inventor : **Bennett, Monte**
**2241 North Iris**
**Escondido, CA 92026 (US)**
Inventor : **Bankert, Charles S.**
**147 Ely Street**
**Oceanside, CA 92054 (US)**
Inventor : **Hui, Henry K.**
**28426 Rancho Grande**
**Laguna Niguel, CA 92677 (US)**
Inventor : **Hahn, Soonkap**
**17110 Cliquot Court**
**Poway, CA 92064 (US)**

(74) Representative : **Mayes, Stuart David et al**
**BOULT, WADE & TENNANT 27 Furnival Street**
**London, EC4A 1PQ (GB)**

(54) **Method of stabilizing a carbon dioxide sensor.**

(57) When exposed to either very low or very high carbon dioxide levels for extended periods of time, carbon dioxide sensors may tend to instability. A stabilizing procedure is accomplished by storing the sensor in an aqueous solution containing at least 2 weight percent carbon dioxide, for from several days to several months. The solution may be prepared in advance, or may be dynamically infused with carbon dioxide to provide the desired carbon dioxide content.

EP 0 481 719 A1

This invention is generally directed to chemical and biochemical analysis of an analyte in a fluid or gaseous mixture, and more specifically relates to methods of stabilizing measurements taken with an intravascular carbon dioxide sensor.

Measurement of acidity (pH) and the tension or partial pressure of carbon dioxide and oxygen in the blood have become important in modern medicine, particularly with regard to determining the respiratory condition of a patient. Although electrodes have been developed which are capable of making such measurements, they are generally of limited use in the medical field. Optical sensors for taking intravascular measurements of acidity, carbon dioxide and oxygen levels in the blood have also been developed, based upon the principle of enclosing a fluorescent indicator within a membrane permeable to the analyte to he measured, coupled to one or more optical fibers for measuring the intensity of fluorescence from the indicator. Since the fluorescence reaction of appropriately chosen indicators is altered according to the level of acidity, carbon dioxide, or oxygen being measured, these sensors allow remote measurement of these parameters when combined with compatible intravascular catheter systems.

A fiber optic chemical sensor may also be used for measuring pH by the use of optical absorbance indicators, such as phenol red, which may he chemically bound in the sensor. In this type of pH sensor, green and red light typically emerge from one optical fiber into the sensor, passing through the dye, to he reflected back through an optical fiber to a detector system. The green light is absorbed by the base form of the indicator, and the, red light is not absorbed by the indicator, so that the red light may be used as an optical reference. The ratio of green to red light can then be measured, and related to pH.

A fluorescent indicator may be used in a similar fashion, with light in one wavelength region being used to excite the fluorescent indicator dye to emit light of a different wavelength. Such optical pH sensors typically include a fluorescent indicator dye, such as fluorescein or hydroxypyrenetrisulfoaic acid (HPTS), placed over the tip of an optical fiber and a membrane cover over the dye which is permeable to the hydronium ions to be measured. The dye fluoresces when exposed to a certain wavelength of light conducted to it by the optical fiber. In practice, a pH sensor is fabricated by immobilizing a pH sensitive dye into a matrix attached to the distal end of the fiber. The dye is typically capable of existing in two forms, an anionic or base form, and a protonated or acid form. The two forms are each excited by a different frequency, but fluoresce at the same frequency, with the output responsive to excitation at the appropriate different frequencies being proportional to the pH of the sample to which the sensor is exposed. In this manner, measurement of the intensity of fluorescence of the indicator dye can be related to pH. A clinically useful range for measuring carbon dioxide as a blood gas parameter has been found to be from about 1.4 weight percent to about 15 weight percent carbon dioxide. Therefore, it is desirable for a carbon dioxide sensor to be accurate and repeatable over at least this range.

It has also been found that carbon dioxide sensors frequently become destabilized when exposed to low carbon dioxide levels, and that a progressive loss of fluorescent intensity occurs in sensors utilizing fluorescent indicators after exposure to high carbon dioxide concentrations. The instability of such fiber optic based carbon dioxide sensors when the sensors are exposed to either very low or very high carbon dioxide levels for prolonged periods of time, such as several days, frequently results in non-specific drift of measurements of carbon dioxide levels. It would therefore be desirable to provide a carbon dioxide blood gas sensor which mitigates this non-specific drift instability.

The present invention is directed toward a new and improved method of stabilizing a carbon dioxide sensor which is adapted for measuring concentrations of carbon dioxide in a fluid. Thus, according to a first aspect of the present invention, there is provided a method of stabilizing a biomedical sensor adapted for measurement of a physiologically significant range of concentrations of an analyte in a fluid, the sensor having a measurable response which is sensitive to changes in an indicator immobilized in said sensor responsive to the concentration of said analyte in said fluid, comprising:

exposing the sensor to a concentration of between 2 weight percent and 100 weight percent of said analyte for a sufficient period of time until the sensor response is relatively stable when exposed to a given concentration of said analyte within said physiologically significant range for prolonged periods of time.

According to a second aspect of the present invention, there is provided a method of stabilizing measurement of the concentration of carbon dioxide in a fluid by a sensor which is sensitive to changes in pH of a buffer immobilized in the sensor, said sensor including at least one dye indicator which when exposed to an energy of excitation exhibits a fluorescence which is altered by carbon dioxide to provide a measurable fluorescence response, comprising:

exposing the sensor to an aqueous solution infused with a gas consisting essentially of from 2 weight percent to 100 weight percent carbon dioxide, with the balance of the gas being inert gas, for a sufficient period of time until said fluorescence response of said indicator is relatively stable when exposed to fluid containing carbon dioxide for prolonged periods of time.

According to a third aspect of the present invention, there is provided a method of stabilizing a carbon

dioxide sensor adapted for measurement of the concentration of an analyte in a fluid, comprising the steps of:

exposing the sensor to a first aqueous solution infused with a relatively high concentration of the analyte to be measured for at least one hour; and

exposing the sensor for an extended period of time to a second aqueous solution infused with a fluid consisting essentially of from 2 weight percent to 100 weight percent of the analyte to be measured, with the balance of the fluid being inert.

The sensor is preferably retained in storage containers containing a solution which has been infused with a gas stream containing from 2 to 100 weight percent carbon dioxide for a period of time varying from several days to several weeks. One currently preferred method is to retain the sensor in a storage container in a prepared solution infused with approximately 8 weight percent carbon dioxide, thus statically maintaining the sensor at a carbon dioxide tension in the midrange of the physiologically significant range. It has been found that storage of the sensor in a solution which has been infused with from 2 to 100 weight percent carbon dioxide eliminates sources of non-specific long term drift that lead to inaccuracy in transduced carbon dioxide content measurements in applications in which the sensor is required to monitor arterial carbon dioxide for prolonged periods of time. This conditioning procedure also facilities faster calibration at the point of use by maintaining the sensor at a physiologically significant carbon dioxide tension immediately prior to use.

These and other objects and advantages of the invention will become apparent from the following detailed description, which illustrates, by way of example, the features of methods in accordance therewith.

Problems of non-specific drift of carbon dioxide blood gas sensors have been observed in both in vitro and in vivo testing for sensor instability. According to a presently preferred embodiment of the method of the present invention, a carbon dioxide sensor is brought into a state of readiness by passive storage in a solution with a carbon dioxide tension in a physiologically significant range. In an alternative preferred embodiment, the sensor may he conditioned by a combination of exposure of the sensor to high carbon dioxide levels in a solution dynamically infused with a gas stream having very high to absolute carbon dioxide tension, followed by passive storage in another solution with a carbon dioxide tension in a physiologically significant range.

The present invention is particularly applicable for stabilizing measurements of the concentration of carbon dioxide in a fluid, such as blood, by a carbon dioxide sensor sensitive to changes in pH of a bicarbonate buffer immobilized in the sensor. A typical sensor incorporates a dye material such as fluorescein in a polymeric matrix, such as silicone, which is permeable to carbon dioxide in the blood. The sensor is typically placed at the end of an optical fiber, which may be inserted into the vasculature of a patient for in vivo blood gas measurements. The sensor is of the type sensitive to changes in pH, and the matrix material is generally soaked in a sodium bicarbonate solution, which serves as a buffer according to the well known equation:

$$CO_2(aq) + H_2O \rightleftharpoons H_2CO_3 \rightleftharpoons H^+ + HCO_3^-$$

According to a first preferred embodiment of the invention, the sensor is typically stored in a sealed container having an aqueous solution which preferably has a relatively high partial pressure of carbon dioxide. The aqueous solution also is preferably osmotically adjusted to approximately match the osmotic pressure of the fluid in which the sensor will eventually be used. The solution is preferably prepared in advance, although it is also possible to infuse the solution with the proper carbon dioxide content after the sensor is placed in the solution.

The aqueous solution preferably should have at least a 2 weight percent carbon dioxide content, and can be prepared by infusing the solution with a gas stream containing from 2 to 100 weight percent carbon dioxide, with the balance being inert gas, such as nitrogen, to infuse the second solution with a physiologically significant carbon dioxide tension. The aqueous solution in which the sensor is to be stored is preferably infused with a gas containing approximately 8 weight percent carbon dioxide, with the balance of the gas being inert gas. The storage solution is also preferably osmotically adjusted to he approximately equivalent to the osmotic strength of the fluid in which the carbon dioxide sensor will eventually he used. The sensor is typically stored in a sealed container with the storage solution for at least one day, and preferably from several days to several months, to condition the sensor for calibration and use.

In another preferred embodiment, the sensor may optionally be preliminarily exposed to a preparatory aqueous solution prior to storage in a second aqueous solution. In this two step process, the sensor is exposed to a preparatory aqueous solution, while a gas stream having a relatively high partial pressure of carbon dioxide, and preferably approximately 100 weight percent carbon dioxide, is periodically or continuously dynamically infused into the solution by bubbling the gas stream in the solution for an hour to a few days as desired.

The sensor is then stored in the second aqueous solution having a 2 weight percent carbon dioxide content

of approximately 2 weight percent or more. The second solution in which the sensor is to be stored is preferably prepared in advance, but may optioaally be dynamically prepared after the sensor has been placed in the solution by infusing a gas stream containing from 2 to 100 weight percent carbon dioxide, with the balance being inert gas, such as nitrogen, to infuse the second solution with a physiologically significant carbon dioxide tension. The physiologically significant range of carbon dioxide concentration for the gas stream is typically from 2 weight percent to 15 weight percent carbon dioxide, with the balance of the gas being inert gas, and the secondary aqueous solution is preferably infused with a gas containing approximately 8 weight percent carbon dioxide, with the balance of the gas being inert gas. The secondary solution is also preferably osmotically adjusted to be approximately equivalent to the osmotic strength of the fluid in which the carbon dioxide sensor will eventually be used. The sensor is then typically stored in a sealed container with the second solution to which the sensor is exposed for at least one day, and preferably from several days to several months, to condition the sensor for calibration and use.

It has been found that carbon dioxide sensors such as those discussed above provide substantially stabilized measurements of carbon dioxide tension for in vitro and in vivo blood gas measurements, not exhibiting the previously observed non-specific drift in measurements of either low or high carbon dioxide levels in solutions for prolonged periods of time. The method also enables faster calibration at the point of use by maintaining the sensor at a physiologically significant carbon dioxide tension.

It should be recognized that other forms of carbon dioxide sensors, such as pH electrodes measuring changes in pH of bicarbonate buffers, or similar buffers, may also be stabilized by the method of the invention.

Thus, it will be apparent to those skilled in the art that the embodiments described provide improved methods of reducing the instability that may occur in carbon dioxide sensors when such sensors are exposed to either very low or very high carbon dioxide levels for extended periods of time. This is accomplished by exposing the sensor to high carbon dioxide levels for a period of time sufficient to allow the sensor to achieve measurement stability thereby decreasing the initial time required to achieve drift stability in comparison with a sensor which has not been exposed in this way.

## Claims

1. A method of stabilizing a biomedical sensor adapted for measurement of a physiologically significant range of concentrations of an analyte in a fluid, the sensor having a measurable response which is sensitive to changes in an indicator immobilized in said sensor responsive to the concentration of said analyte in said fluid, comprising:

   exposing the sensor to a concentration of between 2 weight percent and 100 weight percent of said analyte for a sufficient period of time until the sensor response is relatively stable when exposed to a given concentration of said analyte within said physiologically significant range for prolonged periods of time.

2. The method of Claim 1, wherein said step of exposing said sensor to a concentration of said analyte comprises:

   storing said sensor in a sealed container for a period of at least one day with an aqueous solution containing a fluid consisting of from 2 weight percent to 100 weight percent of the analyte to be measured with the balance of the fluid being inert.

3. The method of Claim 1, wherein said step of exposing said sensor to a concentration of said analyte comprises storing said sensor in a sealed container for a period of at least one day with an aqueous solution dynamically infused with a gas consisting essentially of from 2 weight percent to 100 weight percent carbon dioxide with the balance being inert gas.

4. The method of Claim 2 or Claim 3, wherein said solution is saturated with a gas consisting essentially of 100 weight percent carbon dioxide.

5. The method of Claim 2 or Claim 3, wherein said solution is osmotically adjusted.

6. A method of stabilizing measurement of the concentration of carbon dioxide in a fluid by a sensor which is sensitive to changes in pH of a buffer immobilized in the sensor, said sensor including at least one dye indicator which when exposed to an energy of excitation exhibits a fluorescence which is altered by carbon dioxide to provide a measurable fluorescence response, comprising:

   exposing the sensor to an aqueous solution infused with a gas consisting essentially of from 2

weight percent to 100 weight percent carbon dioxide, with the balance of the gas being inert gas, for a sufficient period of time until said fluorescence response of said indicator is relatively stable when exposed to fluid containing carbon dioxide for prolonged periods of time.

**7.** The method of any of Claims 2, 3 or 6, wherein prior to said step of exposing said sensor in said aqueous solution, said sensor is exposed to a preparatory aqueous solution containing a concentration of at least 2 weight percent carbon dioxide for at least one hour.

**8.** The method of any of Claims 2, 3 or 6, wherein prior to said step of exposing said sensor to the aqueous solution, said sensor is exposed to an aqueous solution dynamically infused with a gas stream consisting essentially of 100 weight percent carbon dioxide for at least one hour.

**9.** The method of Claim 6, wherein said aqueous solution is infused with essentially 100 weight percent carbon dioxide.

**10.** A method of stabilizing a carbon dioxide sensor adapted for measurement of the concentration of an analyte in a fluid, comprising the steps of:

exposing the sensor to a first aqueous solution infused with a relatively high concentration of the analyte to be measured for at least one hour; and

exposing the sensor for an extended period of time to a second aqueous solution infused with a fluid consisting essentially of from 2 weight percent to 100 weight percent of the analyte to be measured, with the balance of the fluid being inert.

**11.** The method of Claim 10, wherein the sensor is exposed to the first aqueous solution while the first aqueous solution is dynamically infused with a fluid stream consisting essentially of 100 weight percent of the analyte to be measured.

**12.** The method of Claim 10, wherein the first solution is osmotically adjusted.

**13.** The method of Claim 10, wherein the second aqueous solution is infused with a fluid consisting essentially of from 2 weight percent to 15 weight percent of the analyte to be measured, with the balance of the fluid being inert.

**14.** The method of Claim 10, wherein the second aqueous solution is infused with a fluid consisting essentially of approximately 8 weight percent analyte to be measured, with the balance of the fluid being inert.

**15.** The method of any of Claims 10 to 14, wherein the analyte to be measured is carbon dioxide.

European Patent Office

# EUROPEAN SEARCH REPORT

Application Number

EP 91 30 9469

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5 ) |
|---|---|---|---|
| X | EP-A-0 270 088 (SUMITOMO ELECTRIC INDUSTRIES)<br>* column 5, line 56 - column 6, line 41 *<br>* column 10, line 24 - line 43; figures 1-4 *<br>* column 13, line 18 - line 47; figures 16-18 *<br>--- | 1,10,15 | A61B5/00<br>G01N33/00<br>G01N21/77 |
| A<br>A | US-A-4 221 567 (J.S. CLARK)<br>* column 5, line 26 - column 6, line 17 *<br>--- | 1-3,10<br>11,15 | |
| A | US-A-4 727 730 (A.A. BOIARSKI ET AL.)<br>* column 3, line 28 - line 51; figures 1,2,6 *<br>* column 8, line 3 - line 8 *<br>----- | 6,15 | |
| | | | TECHNICAL FIELDS SEARCHED (Int. Cl.5 ) |
| | | | A61B<br>G01N |

The present search report has been drawn up for all claims

| Place of search | Date of completion of the search | Examiner |
|---|---|---|
| THE HAGUE | 27 JANUARY 1992 | RIEB K.D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)